Europäisches Patentamt

European Patent Office

Office européen des brevets

(19)

(11) Numéro de publication : **0 234 637 B1**

(12) **FASCICULE DE BREVET EUROPEEN**

(45) Date de publication du fascicule du brevet :
10.07.91 Bulletin 91/28

(51) Int. Cl.⁵ : **C07C 17/34**

(21) Numéro de dépôt : 87200202.7

(22) Date de dépôt : 10.02.87

(54) **Procédé de déshydrochloration en phase liquide d'halogénoalcanes en présence d'un initiateur à base de produit chloré organique.**

(30) Priorité : 20.02.86 BE 216285

(43) Date de publication de la demande :
02.09.87 Bulletin 87/36

(45) Mention de la délivrance du brevet :
10.07.91 Bulletin 91/28

(84) Etats contractants désignés :
AT BE CH DE ES FR GB GR IT LI LU NL SE

(56) Documents cités :
EP-A- 0 133 690
EP-A- 0 172 596
BE-A- 904 251
US-A- 2 593 451

(73) Titulaire : SOLVAY & Cie (Société Anonyme)
Rue du Prince Albert, 33
B-1050 Bruxelles (BE)

(72) Inventeur : Franklin, James
Rue Edouard Olivier, 28
B-1170 Bruxelles (BE)

(74) Mandataire : Nichels, William et al
Solvay & Cie Département de la Propriété
Industrielle Rue de Ransbeek, 310
B-1120 Bruxelles (BE)

## Description

La présente invention concerne un procédé de déshydrochloration en phase liquide d'halogénoalcanes tels que les tétrachloréthanes en présence d'un initiateur à base de produit chloré organique.

Il est connu par le brevet français 947324 que la déshydrochloration pyrolytique d'halogénoalcanes en phase vapeur, notamment du dichloréthane, du tétrachloréthane, du 1,1,2-trichloréthane et du dichloropropane peut être catalysée ou initiée par adjonction de petites quantités de chlore ou d'une substance fournissant du chlore à une température élevée, par exemple de l'hexachloréthane.

Il est également connu par la demande de brevet européen Al-0133690 que la déshydrochloration pyrolytique du 1,2-dichloréthane peut être effectuée en phase gazeuse entre 300 et 600° sous pression atmosphérique ou sous pression plus élevée en présence de composés contenant 3 atomes de carbone et au moins 6 atomes de chlore.

Par ailleurs dans la demande de brevet européenne 0172596, non prépubliée lors du dépôt de la présente demande, on décrit un procédé de déshydrochloration pyrolytique, effectué en phase gazeuse, d'halogénoalcanes en présence d'un initiateur à base de produits organiques chlorés tels qu'un octachorobutène ou du décachlorobutane.

Ces procédés sont opérés en phase gazeuse ou en phase vapeur et présentent tous les inconvénients propres aux réactions pyrolytiques. En effet tous les hydrocarbures chlorés connus comme initiateurs ne conduisent en delà de 350° qu'à des taux de pyrolyse peu élevés. Pour augmenter ces taux de pyrolyse, on est amené à travailler à haute température, ce qui augmente en conséquence les dépenses d'énergie et donne lieu à la formation de sous-produits et de coke ; ce dernier se dépose sur les parois du réacteur de pyrolyse nécessitant ainsi des arrêts périodiques pour son nettoyage.

Enfin, le brevet Etats-Unis 2,593,451 protège une invention dans laquelle on prépare des chloréthanes insaturés par déshydrochloration des polychloréthanes saturés correspondants, ayant au moins trois atomes de chlore, par un procédé dans lequel on opère en phase liquide anhydre en présence de chlorure ferrique.

A ce jour il n'existe donc pas un procédé de déshydrochloration d'halogénoalcanes effectué en phase liquide en présence d'initiateurs à base de produits chlorés organiques qui ne présenterait plus les inconvénients des procédés réalisés en phase gazeuse.

La présente invention a pour but de remédier à cette carence en fournissant un nouveau procédé pour la déshydrochloration en phase liquide d'halogénoalcanes en présence d'initiateurs à base de produits chlorés organiques.

L'invention concerne à cet effet un procédé de déshydrochloration d'halogénoalcanes en présence d'un initiateur à base de produit chloré organique qui est effectué en phase liquide dans des zones de température comprises entre 150 et 350°C et à des pressions comprises entre la pression atmosphérique et 20,4 bars et à l'intervention d'un initiateur constitué principalement de décachlorobutane ou d'un octachlorobutène tel que l'octachlorobutène-1 ou d'un mélange de ces produits. De préférence, l'initiateur à base de produit chloré résulte de la chloration additive d'hexachlorobutadiène-1,3.

Par produit chloré, on entend le ou les produits résultant de la chloration additive d'hexachlorobutadiène-1,3 pouvant être obtenu de manière connue en soi, par exemple par photochloration ou par chloration en phase liquide catalysée par le fer. Quand la chloration additive de l'hexachlorobutadiène conduit à la formation de mélanges de produits, ces mélanges contiennent, en plus des produits principaux cités plus haut, un peu d'hexachloréthane et éventuellement de l'hexachlorobutadiène-1,3 non transformé, ainsi qu'une faible proportion de divers autres produits. Quand la chloration additive de l'hexachlorobutadiène conduit à la formation d'un produit chloré unique, il s'agit généralement d'un octachlorobutène tel que l'octachlorobutène-1 ou du décachlorobutane.

L'obtention d'un mélange de produits ou d'un produit chloré unique dépend notamment du degré de chloration de l'hexachlorobutadiène, de la température et de la nature du catalyseur.

Il est toutefois évident que par mélange de produits chlorés résultant de la chloration additive d'hexachlorobutadiène-1,3, on entend non seulement les mélanges issus directement de la chloration mais également les mélanges ayant, après la chloration, subi certaines modifications, telles que celles résultant d'opérations de distillation ou de rectification ainsi que d'opérations de cristallisation, permettant d'obtenir un enrichissement en l'un ou l'autre des constituants du mélange.

Enfin en ce qui concerne le produit chloré unique issu généralemnt de la chloration additive de l'hexachlorobutadiène-1,3 pouvant être mis en oeuvre dans le procédé de l'invention, il est également évident que son origine n'est pas critique en sol pour le procédé de déshydrohalogénation en phase liquide de l'invention et que ce produit unique peut avoir comme origine toute autre matière première que l'hexachlorobutadiène-1,3.

A titre d'exemple, on donne ci-après la composition, en g/kg, de deux mélanges bruts résultant de la photochloration de l'hexachlorbutadiène-1,3 :

| | Mélange I | Mélange II |
|---|---|---|
| Octachlorobutène-1 | 368 | 202 |
| Décachlorobutane | 438 | 609 |
| Hexachloréthane | 68 | 111 |
| Hexachlorobutadiène-1,3 | 119 | 23 |
| Autres produits, non identifiés | 7 | 55 |

Ces mélanges bruts de produits ainsi que d'ailleurs certains constituants de ces mélanges peuvent être utilisés dans le procédé selon l'invention, en une quantité pondérale comprise habituellement entre 0,1 et 20% et de préférence entre 0,2 et 10% par rapport à l'halogénoalcane soumis à la déshydrochloration en phase liquide. De bons résultats ont été obtenus avec des quantités pondérales, du Mélange I et du Mélange II, voisines de 4% par rapport à l'halogénoalcane soumis à la déshydrochloration pyrolytique.

Les initiateurs à base de produit chloré mis en oeuvre dans le procédé de l'invention ont un effet accélérateur très puissant dans des zones de température de la phase liquide comprises entre 150 et 350°C et de préférence entre 175° et 300°C. Ainsi dans le cas de la déshydrochloration en phase liquide du 1,1,2-tétrachloréthane sous pression, on obtient déjà des taux de déshydrochloration intéressants avec les mélanges bruts de la chloration additive de l'hexachlorbutadiène-1,3 mis en oeuvre à raison de 4% en poids par rapport au 1,1,2-tétrachloréthane à des températures de 200°C. Les taux de déshydrochloration augmentent bien entendu avec l'élévation de la température mais au prix de l'apparition de réactions secondaires qui donnent lieu à la formation de sous-produits indésirables. Le procédé selon l'invention permet donc de choisir une solution de compromis au niveau de la température de la déshydrochloration.

Un avantage important du procédé selon l'invention réside dès lors dans le fait qu'il peut être appliqué dans une phase réactionnelle différente et requérant des températures inférieures à celles que l'on peut utiliser dans les procédés de l'art antérieur. Des températures comprises entre 150 et 350°C ont donné de bons résultats pour les différents halogénoalcanes étudiés.

Un autre avantage du procédé selon l'invention réside dans le fait qu'il peut être exploité soit à la pression atmosphérique soit sous une pression supérieure. De préférence on opère sous pression atmosphérique ou sous une pression modérée.

Par pression modérée, on entend des pressions inférieures à 20 atmosphères (20,4 bars). De bons résultats ont été obtenus à des pressions comprises entre 1 et 15 bars.

Le procédé de déshydrochloration en phase liquide d'halogénoalcanes selon l'invention peut être appliqué à un grand nombre de réactions. On peut notamment, sans aucune intention limitative, mentionner les applications suivantes :
 – fabrication de chlorure de vinyle à partir de 1,2-dichloréthane
 – fabrication de chlorure de vinylidène et de 1,2-dichloréthylène cis et trans à partir de 1,1,2-trichloréthane
 – fabrication de trichloréthylène à partir de 1,1,2,2-tétrachlorétane ou de 1,1,1,2-tétrachloréthane.

Selon les cas, il convient d'appliquer les conditions de pression et de température adéquates adaptées à la nature des halogénoalcanes mis en oeuvre de façon à opérer dans la phase liquide tout en maintenant les conditions de températures auxquelles l'initiateur à base de produit chloré exerce un effet optimal.

On a également constaté qu'il peut être souhaitable dans certains cas, afin de minimiser la surchauffe du mélange réactionnel, d'exécuter la réaction de déshydrochloration en phase liquide en présence d'additifs qui agissent comme diluants mais qui sont inertes vis-à-vis des réactifs et des initiateurs intervenant dans la réaction. Comme additifs on utilise de préférence des dérivés chlorés aliphatiques tel que le tétrachlorure de carbone ou l'hexachlorbutadiène-1,3 ou des produits inorganiques tels que le chlorure d'hydrogène ou l'azote. De préférence on opère avec le tétrachlorure de carbone ou l'hexachlorobutadiène-1,3.

En général les additifs organiques halogénés sont ajoutés au milieu réactionnel à raison de 1 à 25 moles par mole d'halogénoalcane mis en oeuvre.

Le procédé selon l'invention peut être réalisé dans tout appareillage ou tout réacteur permettant de réunir les conditions opératoires décrites ci-avant.

Les exemples suivants sont donnés à titre explicatif du procédé selon l'invention.

Les exemples 1 et 2R sont réalisés dans un appareillage de laboratoire en pyrex comprenant un ballon de 250 cm³, surmonté d'un condenseur à reflux refroidi à l'eau glacée. Le sommet du condenseur est relié à deux barbotteurs placés en série. Le premier contient 50 cm³ de $CCl_4$ permettant d'absorber des organiques éventuellement présents dans les gaz sortant du condenseur ; le second contient 100 cm³ d'eau, destinée à abattre le chlorure d'hydrogène formé lors de la déshydrochloration.

EP 0 234 637 B1

Un débit d'azote d'environ 12 litres normaux par heure, est injecté dans le ballon, afin d'entraîner le HCl formé vers le barbotteur à eau, où il est dosé en fonction du temps.

## Exemple 1

On introduit dans le ballon de 250 cm³, 20 g de 1,1,1,2-tétrachloréthane et 180 g d'hexachloro-1,3-butadiène. Ce dernier sert de tiers solvant lourd, permettant d'atteindre la température désirée, tout en opérant à la pression atmosphérique.

On ajoute ensuite 1,10 g d'initiateur (Mélange I), ce qui correspond à 2% mol ($C_4 Cl_8$ + $C_4Cl_{10}$) par rapport au 1,1,1,2-$C_2H_2Cl_4$ mis en oeuvre.

On porte le mélange réactionnel à l'ébullition. Il faut environ 15 minutes pour atteindre la température de reflux, qui est de 190°C.

La quantité de HCl dosé dans le barbotteur à eau correspond à des conversions du 1,1,1,2-$C_2H_2Cl_4$ en trichloréthylène de 77 et 93% après respectivement 1 et 2 heures de réaction.

Le seul produit organique retrouvé en fin de réaction, outre le 1,1,1,2-$C_2H_2Cl_4$ non transformé, le tiers solvant, l'initiateur et des produits de décomposition, est le trichloréthylène.

## Exemple 2R

Cet essai est effectué dans des conditions identiques à celles de l'exemple 1, sauf que l'on opère sans addition d'initiateur. La quantité de HCl dosé correspond à des conversions du 1,1,1,2-$C_2H_2Cl_4$ de seulement 16 et 39% après respectivement 1 et 2 heures de réaction.

## Exemple 3

Dans cet exemple les réactions sont effectuées dans un autoclave agité en Hastelloy C, d'un volume de 1 litre, chauffé électriquement.

500 cm³ (793 g) de 1,1,2,2-tétrachloréthane sont introduits dans l'autoclave et portés à l'ébullition (200°C) sous une pression absolue de 3,7 bar, régulée à l'aide d'une vanne automatique reliée à la phase gazeuse du réacteur.

On introduit rapidement, à l'aide d'une pompe doseuse (durée environ 2 minutes), 32,7 g d'initiateur (Mélange II) dissous dans 72 g de 1,1,2,2-tétrachloréthane.

Le chlorure d'hydrogène dégagé lors de la déshydrochloration ainsi que les organiques présents en phase vapeur sont entraînés, sortent par la vanne de régulation de la pression, sont détendus à la pression atmosphérique et sont dirigés vers un condenseur refroidi à l'eau froide et un séparateur gaz/liquide. Le liquide condensé est repris par une pompe doseuse et réintroduit dans l'autoclave. Les gaz non condensés sont dirigés vers un scrubber arrosé par de l'eau, afin d'abattre le HCl, qui est dosé périodiquement dans la phase aqueuse recueillie au bas du scrubber, ce qui permet de suivre l'avancement de la réaction de déshydrochloration.

La quantité d'initiateur mis en oeuvre correspond à 1,83% mol de ($C_4Cl_8$ + $C_4Cl_{10}$) par rapport au 1,1,2,2-tétrachloréthane initial. Après 40 minutes de réaction, la quantité de HCl dégagé correspond à une conversion du 1,1,2,2-tétrachloréthane en trichloréthylène de 16% et la vitesse initiale de dégagement de HCl est de 0,08 mol/litre 1,1,2,2-tétrachloréthane.min.

## Exemple 4R

Cet exemple est réalisé dans les mêmes conditions opératoires que l'exemple 3 ci-dessus mis sans addition d'initiateur.

Après 40 minutes de réaction, sans initiateur, la quantité de HCl dégagé correspond à une conversion du 1,1,2,2-tétrachloréthane de seulement 1,8%. La vitesse initiale n'atteint que 0,004 mol HCl/litre 1,1,2,2-tétrachloréthane.min.

## Exemple 5 et 6R

Les essais sont effectués dans le même appareillage que l'exemple 3.

213 cm³ (337 g) de 1,1,2,2-$C_2H_2Cl_4$ sont introduits dans l'autoclave et portés à l'ébullition (200°C) sous une pression absolue de 3,7 bar. A l'aide de la pompe doseuse, 35,1 g d'initiateur (Mélange II) dissous dans 32 g de 1,1,2,2-tétrachloréthane sont introduits à un débit régulier sur un laps de temps d'une heure. Au total on introduit 4,6% mol de ($C_4Cl_8$ + $C_4Cl_{10}$) par rapport au 1,1,2,2-tétrachloréthane mis en oeuvre. Lorsque tout

4

l'initiateur est introduit, la quantité de HCl dégagé correspond à une conversion du 1,1,2,2-tétrachloréthane en trichloréthylène de 34% (exemple 5).

En opérant sans ajout d'initiateur (exemple 6R) la conversion n'atteint que 2,5% après 1 heure.

## Revendications

1. Procédé de déshydrochloration d'halogénoalcanes en présence d'un initiateur à base de produit chloré organique caractérisé en ce qu'il est effectué en phase liquide dans des zones de températures comprises entre 150 et 350°C et à des pressions comprises entre la pression atmosphérique et 20,4 bars et à l'intervention d'un initiateur constitué principalement de décachlorobutane ou d'un octachlorobutène tel que l'octachlorobutène-1 ou d'un mélange de ces produits.

2. Procédé suivant la revendication 1 caractérisé en ce que l'initiateur résulte de la chloration additive de l'hexachlorobutadiène-1,3.

3. Procédé suivant les revendications 1 ou 2 caractérisé en ce qu'on utilise l'initiateur en une quantité de l'ordre de 4% du poids de l'halogénure mis en oeuvre.

4. Procédé suivant l'une des revendications 1 à 3 caractérisé en ce qu'il est appliqué à la fabrication de chlorure de vinyle par déshydrochloration en phase liquide de 1,2-dichloréthane.

5. Procédé suivant l'une des revendications 1 à 3 caractérisé en ce qu'il est appliqué à la fabrication de chlorure de vinylidène par déshydrochloration en phase liquide de 1,1,2-trichloréthane.

6. Procédé suivant l'une des revendications 1 à 3 caractérisé en ce qu'il est appliqué à la fabrication de trichloréthylène par déshydrochloration en phase liquide de 1,1,2,2-tétrachloréthane ou de 1,1,1,2-tétrachloréthane.

## Claims

1. Process for dehydrochlorination of haloalkanes in the presence of an initiator based on an organic chlorinated product, characterised in that it is performed in the liquid phase in temperature ranges between 150 and 350°C and at pressures between atmospheric pressure and 20.4 bars, and with the participation of an initiator consisting mainly of decachlorobutane or of an octachlorobutene such as octachloro-1-butene or of a mixture of these products.

2. Process according to Claim 1, characterised in that the initiator results from the additive chlorination of hexachloro-1,3-butadiene.

3. Process according to Claim 1 or 2, characterised in that the initiator is used in an amount of the order of 4% of the weight of the halide used.

4. Process according to one of Claims 1 to 3, characterized in that it is applied to the manufacture of vinyl chloride by liquid phase dehydrochlorination of 1,2-dichloroethane.

5. Process according to one of Claims 1 to 3, characterized in that it is applied to the manufacture of vinylidene chloride by liquid phase dehydrochlorination of 1,1,2-trichloroethane.

6. Process according to one of Claims 1 to 3, characterized in that it is applied to the manufacture of trichloroethylene by liquid phase dehydrochlorination of 1,1,2,2-tetrachloroethane or 1,1,1,2-tetrachloroethane.

## Patentansprüche

1. Verfahren zur Dehydrochlorierung von Halogenalkanen in Gegenwart eines Initiators auf der Basis von organischen chlorierten Produkten, dadurch gekennzeichnet, daß es in der Flüssigphase in Temperaturbereichen zwischen 150 und 350°C und Drucken zwischen dem atmosphärischen Druck und 20,4 Bar und mittels eines Initiators, der im wesentlichen aus Decachlorbutan oder einem Octachlorbuten, wie dem Octachlorbuten-1 oder einer Mischung dieser Produkte gebildet ist, durchgeführt wird.

2. Verfahren nach Anspruch 1, dadurch gekennzeichnet, daß der Initiator aus der zusätzlichen Chlorierung des Hexachlorbutadien1,3 stammt.

3. Verfahren nach den Ansprüchen 1 oder 2, dadurch gekennzeichnet, daß der Initiator in einer Menge von 4 Gew.-% des eingesetzten Halogenids verwendet wird.

4. Verfahren nach einem der Ansprüche 1 bis 3, dadurch gekennzeichnet, daß es zur Herstellung von Vinylchlorid durch Dehydrochlorierung in der Flüssigphase von 1,2-Dichlorethan verwendet wird.

5. Verfahren nach einem der Ansprüche 1 bis 3, dadurch gekennzeichnet, daß es zur Herstellung von Vinylidenchlorid durch Dehydrochlorierung in der Flüssigphase von 1,1,2-Trichlorethan verwendet wird.

6. Verfahren nach einem der Ansprüche 1 bis 3, dadurch gekennzeichnet, daß es zur Herstellung von Trichlorethylen durch Dehydrochlorierung in der Flüssigphase von 1,1,2,2-Tetrachlorethan oder 1,1,1,2-Tetrachlorethan verwendet wird.